# EUROPEAN PATENT APPLICATION

(11) **EP 2 275 146 A1**
(43) Date of publication of application: **19.01.2011**
(21) Application number: 09723868.7
(22) Date of filing: 24.03.2009
(51) Int. Cl.: A61L 2/14, A61L 2/24

(54) **STERILIZER AND STERILIZATION METHOD**

(30) Priority: 26.03.2008 JP 2008081825
(71) Applicant: Saian Corporation, Wakayama-shi Wakayama 6408550 (JP)
(72) Inventor: ARAI, Kiyotaka, Wakayama-shi Wakayama 640-8550 (JP); HIROSE, Tomoyuki, Wakayama-shi Wakayama 640-8550 (JP); MIKE, Masaaki, Wakayama-shi Wakayama 640-8550 (JP); IWASAKI, Ryuichi, Wakayama-shi Wakayama 640-8550 (JP)
(74) Representative: Müller-Boré & Partner Patentanwälte
(86) International application number: PCT/JP2009/055838
(87) International publication number: WO 2009/119593

(57) **Abstract**

A sterilizer includes: a supply source for a sterilizing agent; a first sterilization chamber and a second sterilization chamber each adapted to be filled with the sterilizing agent while placing an object therein so as to subject the object to a sterilization treatment; a first pipe line connecting the supply source and each of the first sterilization chamber and the second sterilization chamber; a second pipe line connecting the first sterilization chamber and the second sterilization chamber; and a supply mechanism adapted to allow a residual part of the sterilizing agent used for the sterilization treatment in the first sterilization chamber to be introduced into the second sterilization chamber via the second pipe line.

## Description

### TECHNICAL FIELD

The present invention relates to an apparatus and method for subjecting an object, such as a medical instrument, to a sterilization treatment.

### BACKGROUND ART

In a sterilizer for subjecting an object to a sterilization treatment, for example, when performing parallel sterilization experiments in biotechnology fields, or when subjecting medical instruments or the like to a sterilization treatment in small lots, it is desired to prepare a plurality of sterilization chambers and perform a sterilization treatment in each of the chamber individually. A conventional technique for meeting such a need is proposed in JP 6-7857B.

A sterilizer in the JP 6-7857B comprises a plurality of sterilization chambers, wherein each of a charge-system pipe and a discharge-system pipe is branched in parallel with respect to respective ones of the sterilization chambers. A sterilization treatment can be performed in each of the sterilization chambers individually by controlling a damper and a valve appropriately installed in the pipes. It is also shown that a gas after being used for the sterilization treatment is reused by circulating it to a sterilizing gas generator.

However, the sterilizer in above Patent Document is an apparatus for use in biotechnologies, and thereby a time period required for the sterilization treatment is hot particularly considered because an experiment cycle is long.

### DISCLOSURE OF THE INVENTION

It is an object of the present invention to provide a sterilizer and method capable of shortening a time period required for a sterilization treatment.

A sterilizer according to one aspect of the present invention which achieves the above object includes: a supply source for a sterilizing agent; a first sterilization chamber and a second sterilization chamber each adapted to be filled with the sterilizing agent while placing an object therein so as to subject the object to a sterilization treatment; a first pipe line connecting the supply source and each of the first sterilization chamber and the second sterilization chamber; a second pipe line connecting the first sterilization chamber and the second sterilization chamber; and a supply mechanism adapted to allow a residual part of the sterilizing agent used for the sterilization treatment in the first sterilization chamber to be introduced into the second sterilization chamber via the second pipe line.

In the above sterilizer, after the sterilization treatment is performed in the first sterilization chamber, a residual sterilizing agent in the first sterilization chamber is introduced into the second sterilization chamber, so that a time period required for filling the second sterilization chamber with a sterilizing agent can be shortened. Preferably, the sterilizing-gas supply source is adapted to form a sterilizing agent by a plasma reaction.

These and other objects, features and advantages of the invention will become more apparent upon reading the following detailed description along with the accompanying drawings.

### BRIEF DESCRIPTION OF THE DRAWINGS

FIG. 1 is a block diagram showing a sterilizer according to a first embodiment of the present invention.
FIG. 2 is a flowchart showing an operation of the sterilizer according to the first embodiment.
FIG. 3 is a block diagram showing a sterilizer according to a second embodiment of the present invention.
FIG. 4 is a block diagram schematically showing a structure of a plasma engine.
FIG. 5 is a sectional view showing a plasma nozzle in a state after it is attached to a waveguide.
FIG. 6 is a time chart showing an operation of the sterilizer according to the second embodiment.
FIG. 7 is a tabular diagram showing a control state of solenoid valves and pumps.
FIG. 8 is a block diagram showing a sterilizer according to a third embodiment of the present invention.
FIG. 9 is a diagram for explaining a treatment process in the sterilizer according to the third embodiment.

### BEST MODE FOR CARRYING OUT THE INVENTION

### [FIRST EMBODIMENT]

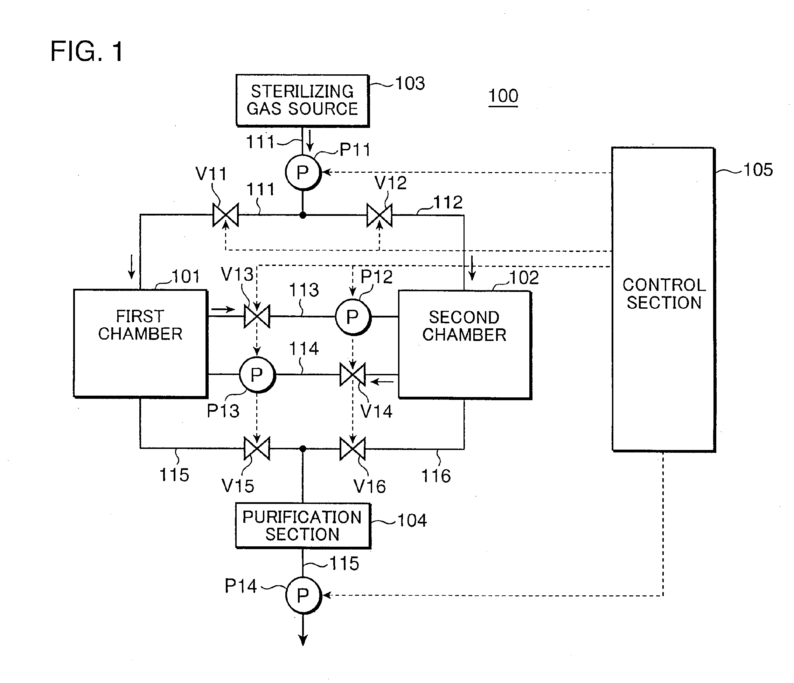

FIG. 1 is a block diagram showing a sterilizer 100 according to a first embodiment of the present invention. The sterilizer 100 is an apparatus which is aimed, for example, at a medical instrument such as a surgical knife, a forceps or a catheter, or a food wrapper such as a wrapping sheet, a tray or a bottle, and designed to allow a sterilizing agent to act on such an object so as to subject the object to a sterilization treatment.

The sterilizer 100 includes: a plurality of units including a first chamber 101 (first sterilization chamber), a second chamber 102 (second sterilization chamber), a sterilizing gas source 103 (supply source for a sterilizing agent) and a purification section 104; first to sixth pipes 111 to 116 interconnecting the units; first to sixth solenoid valves V11 to V16 and first to fourth pumps P11 to P14 which are installed in respective suitable positions of the pipes; and a control section 105 for performing an electrical control for the sterilizer 100.

The first chamber 101 and the second chamber 102 are chambers which are substantially identical to each other, and each of which provides a hermetically closed space for receiving therein an object, and may have a pressure-resistant structure made, for example, of stainless steel. Although illustration is omitted, each of the two chambers 101, 102 has a door for allowing an object to be carried therein/out thereof, and a treatment tray provided thereinside to allow an object to be placed thereon. Further, various sensor elements, such as a concentration sensor for measuring a concentration of a sterilizing agent, and a pressure sensor for detecting an internal pressure of the chamber, are installed inside each of the two chambers 101, 102.

The sterilizing gas source 103 is operable to supply a sterilizing gas (sterilizing agent), such as ethylene oxide gas, nitrogen oxide gas or nitrogen dioxide gas, to each of the first chamber 101 and the second chamber 102. For example, the sterilizing gas source 103 may be a sterilizing-gas supply cylinder, or a device adapted to subject a raw material gas to a specific reaction treatment (e.g., plasma reaction treatment) to form a sterilizing gas.

The purification section 104 is operable to purify a sterilizing gas and a reaction product left after the sterilization treatment of the object in each of the first chamber 101 and the second chamber 102. The control section 105 comprises a CPU (Central Processing Unit), wherein it is operable, based on a given program, to perform an operational control for the first to fourth pumps P11 to P14, and an opening/closing control for the first to sixth solenoid valves V11 to V16.

The sterilizing gas source 103 and the first chamber 101 are connected via the first pipe 111 (a part of a first pipe line), and the first pump P11 (a introduction mechanism) and the first solenoid value V11 are installed in the first pipe 111. The second pipe 112 (a part of the first pipe line) is branched from the first pipe 111 at a position between the first pump P11 and the first solenoid value V11. The second solenoid valve V12 is installed in the second pipe 112. The second chamber 102 is connected to the sterilizing gas source 103 via the second pipe 112 and an upstream portion of the first pipe 111.

A supply mechanism is provided between the first chamber 101 and the second chamber 102 to allow a sterilizing gas remaining after performing the sterilizing treatment in one of the chambers (residual sterilizing gas) to be introduced the other chamber. In the first embodiment, a the third pipe 113 and the fourth pipe 114 (second pipe line) connecting the first chamber 101 and the second chamber 102, the second pump P12 and the third solenoid valve V13 each installed in the third pipe 113, and the third pump P13 and fourth third solenoid valve V14 each installed in the fourth pipe 114, are provided as the supply mechanism.

The second pump P12 is a pump for generating a gas flow causing the gas in the first chamber 101 to be sent into the second chamber 102 through the third pipe 113. The third pump P13 is a pump for generating a gas flow causing the gas in the second chamber 102 to be sent into the first chamber 101 through the fourth pipe 114. In cases where the gas is transferred by means of a pressure difference between the first chamber 101 and the second chamber 102, the second and third pumps P12, P13 may be omitted.

The fifth pipe 115 for discharge is led from the first chamber 101. The fifth solenoid valve V15, the purification section 104, and the fourth pump P14 for discharge, are installed in the fifth pipe 115 in this order from an upstream side. Further, the sixth pipe 116 for discharge is led from the second chamber 102. The sixth solenoid valve V16 is installed in the sixth pipe 116, and a downstream end of the sixth pipe 116 is joined with the fifth pipe 115 at a position between the fifth solenoid valve V15 and the purification section 104.

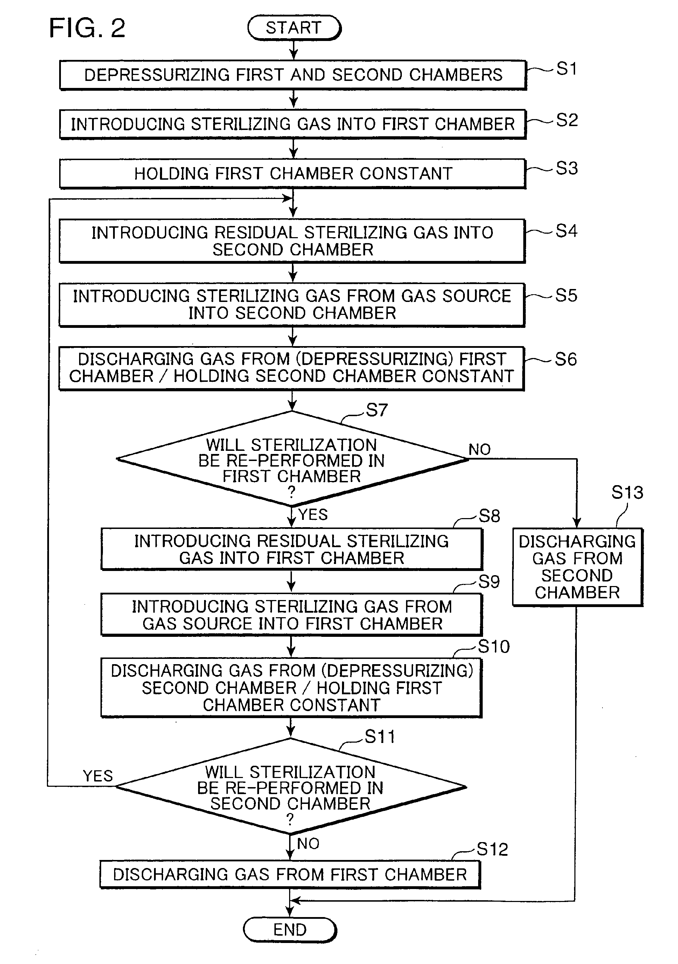

An operation of the sterilizer 100 constructed as above will be described based on the flowchart illustrated in FIG. 2. Firstly, under a condition that an object is placed in each of the first chamber 101 and the second chamber 102, the two chambers 101, 102 are depressurized (Step S1). During this step, the control section 105 operates to open the fifth and sixth solenoid valves V15, V16 and close the remaining solenoid valves, and then operates to activate the third pump P13 to vacuumize the two chambers 101, 102. This allows the object in each of the chambers to be brought into a dry state. This vacuuming may be omitted depending on a type of object.

Secondly, the control section 105 operates to open the first solenoid valve V11 and close all of the remaining solenoid valves, and then operates to activate the first pump P11. This allows a sterilizing gas to be introduced from the sterilizing gas source 103 into the first chamber 101 (Step S2). The supply of the sterilizing gas will be continued until an inside of the first chamber 101 has atmospheric or ambient pressure. After that, the control section 105 operates to close the first solenoid valve V11, and hold this state only for a given time period required for sterilization (Step S3).

Subsequently, the step of allowing a residual part of the sterilizing gas used for the sterilization in the first chamber 101 to be introduced into the second chamber 102 (Step S4) is executed. For this step, the control section 105 operates to open only the third solenoid valve V13 and activate the second pump P12. This allows the residual sterilizing gas to be introduced into the second chamber 102 at once, in combination with a pressure difference between the first chamber 101 and the second chamber 102. This state will be continued until respective pressures of the first chamber 101 and the second chamber 102 are counterbalanced. Through this operation, about 50% of sterilizing gas in terms of a required amount can be introduced into the second chamber 102, within a short time period.

After that, the control section 105 operates to open the second solenoid valve V12 and close all of the remaining solenoid valves, and then operates to activate the first pump P11. This allows a sterilizing gas to be introduced from the sterilizing gas source 103 into the second chamber 102 (Step S5). In this step, a gas-introduction time period can be shortened because about 50% of the required sterilizing gas has already been introduced in the second chamber 102 through the previous Step S4. In the Step S4, the second pump P12 may be continuously activated after the pressure counterbalancing to send a larger amount of sterilizing gas into the second chamber 102. In this case, the gas-introduction time period in the Step S5 can be further shortened.

Subsequently, discharge of gas remaining in the first chamber 101, and the sterilization treatment of the object in the second chamber 102, are performed in parallel or concurrently (Step S6). During this step, the control section 105 operates to close at least the second, third, fourth and sixth valve V12, V13, V14, V16 to keep the second chamber 102 in the hermetically closed state only for a given time period required for sterilization. Concurrently, the control section 105 operates to open the fifth solenoid valve V15 and then activate the fourth pump P14 to discharge a sterilizing gas remaining in the first chamber 101, while detoxifying or purifying the gas through the purification section 104. Then, the sterilized object is taken out of the first chamber 101.

After that, it is determined whether a setting for re-performing the sterilization treatment in the first chamber 101 is made (Step S7). If the re-performance is scheduled (YES in the Step S7), the step of allowing a residual sterilizing gas in the second chamber 102 to be introduced into the first chamber 101 is executed (Step S8). In this case, in advance of the Step S8, an operator places a new object in the first chamber 101, and then the same depressurization treatment as that in the Step S1 is performed for the first chamber 101.

In the Step S8, the control section 105 operates to open only the solenoid valve V14 and activate the third pump P13. This allows the residual sterilizing gas in the second chamber 102 to be introduced into the first chamber 101. This introduction operation is a reverse operation to that in the Step S4, so that, through this operation, about 50% of a required amount can be introduced into the first chamber 101, within a short time period.

After that, the control section 105 operates to open the first solenoid valve V11 and close all of the remaining solenoid valves, and then activate the first pump P11. This allows a sterilizing gas to be introduced from the sterilizing gas source 103 into the first chamber 101 (Step 9). In this step, a gas-introduction time period can be shortened because about 50% of the required sterilizing gas has already been introduced in the first chamber 101 through the previous Step S8.

Subsequently, discharge of gas remaining in the second chamber 102, and the sterilization treatment of the object in the first chamber 101, are concurrently performed (Step S10). During this step, the control section 105 operates to close at least the first, third, fourth and fifth valve V11, V13, V14, V15 to keep the first chamber 101 in the hermetically closed state only for a given time period required for sterilization. Concurrently, the control section 105 operates to open the sixth solenoid valve V16 and then activate the fourth pump P14 to discharge a sterilizing gas remaining in the second chamber 102, while detoxifying or purifying the gas through the purification section 104. Then, the sterilized object is taken out of the second chamber 102.

After that, it is determined whether a setting for re-performing the sterilization treatment in the second chamber 102 is made, i.e., whether the sterilization treatment of an object is subsequently scheduled (Step S11). If the re-performance is scheduled (YES in the Step S11), the routine is returned to the Step S4 to repeat the above process. Otherwise, if the re-performance is not scheduled (NO in the Step S11), after completing the sterilization in the first chamber 101, the control section 105 operates to open the fifth solenoid valve V15 and then activate the fourth pump 14 to discharge gas remaining in the first chamber 101 (Step S12), and then operates to terminate the process. The same applies to a case where the re-performance of the sterilization treatment in the first chamber 101 is not scheduled in the Step S7 (NO in the Step S7). In this case, the control section 105 operates to open the sixth solenoid valve V16 and then activate the fourth pump 14 to discharge gas remaining in the second chamber 102 (Step S13), and then operates to terminate the process.

In the sterilizer 100 according to the first embodiment as described above, a residual sterilizing gas after the sterilizing treatment is exchanged between the first chamber 101 and the second chamber 102, so that a time period required for introducing a sterilizing gas into each of the chambers can be shortened to improve operating efficiency of the sterilization treatment.

### [SECOND EMBODIMENT]

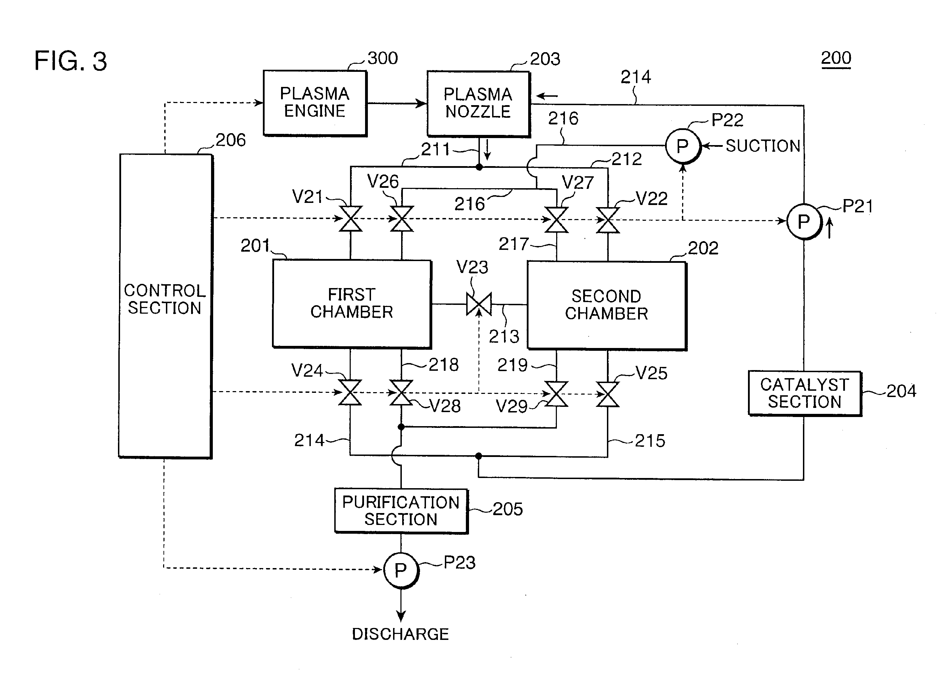

FIG. 3 is a block diagram showing a sterilizer 200 according to a second embodiment of the present invention. The sterilizer 200 is an apparatus which is aimed at a medical instrument, a food wrapper, or the like, and designed to allow nitrogen dioxide (NO₂) gas as a sterilizing agent to act on such an object so as to subject the object to a sterilization treatment. The second embodiment shows an example where a plasma reaction is employed to form NO₂ gas.

The sterilizer 200 includes: a plurality of units including a first chamber 201 (first sterilization chamber), a second chamber 202 (second sterilization chamber), a plasma nozzle 203 (supply source for a sterilizing agent/sterilizing gas-forming section), a catalyst section 204 and a purification section 205; first to ninth pipes 211 to 219 interconnecting the units; first to ninth solenoid valves V21 to V29 (the first and fourth solenoid valves V21, V24 are a first shutoff device; the second and fifth solenoid valves V22,V25 are a second shutoff device; the third solenoid valve V23 is a third shutoff device) and first to third pumps P21 to P23 (the first pump P21 is a circulation mechanism) which are installed in respective suitable positions of the pipes; a control section 205 for performing an electrical control for the sterilizer 200; and a plasma engine 300.

The first chamber 201 and the second chamber 202 are chambers which are substantially identical to each other, and each of which provides a hermetically closed space for receiving therein an object, and may have a pressure-resistant structure made, for example, of stainless steel. Although illustration is omitted, each of the two chambers 201, 202 has a door for allowing an object to be carried therein/out thereof, and a treatment tray provided thereinside to allow an object to be placed thereon. Further, although illustration is omitted, various sensor elements, such as a concentration sensor for measuring a concentration of a sterilizing agent, and a pressure sensor for detecting an internal pressure of the chamber, are installed inside each of the two chambers 201, 202.

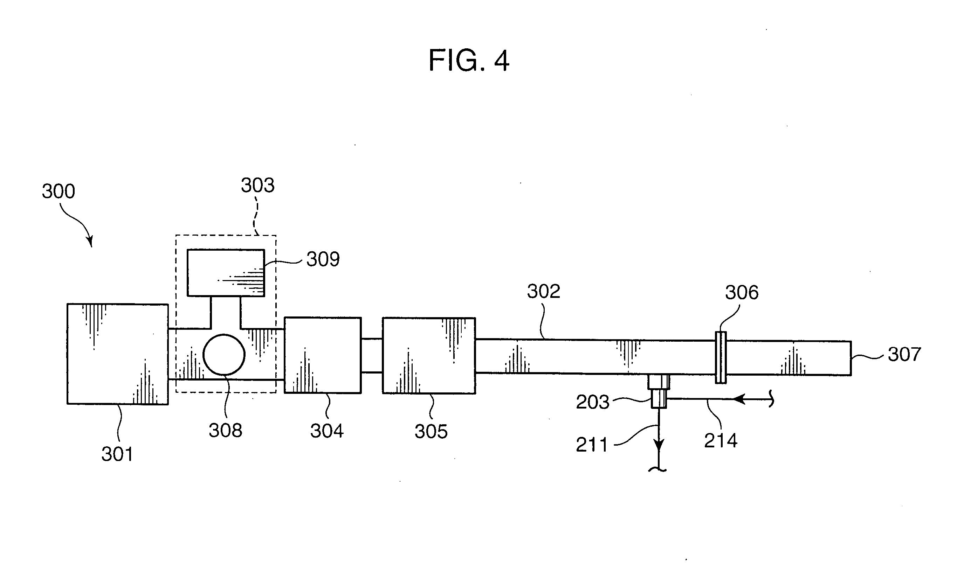

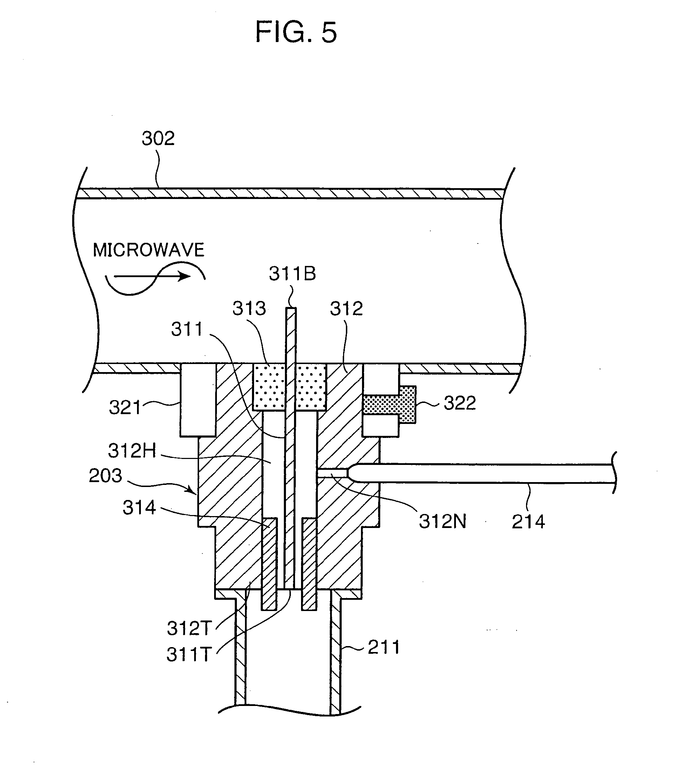

The plasma nozzle 203 is adapted to provide a concentrated electric field region for generating plasma (ionized gas). The plasma nozzle 203 has a plasma generation space, wherein it is operable to plasmatize (ionize) a raw material gas (in this embodiment, air) containing nitrogen and oxygen and passing through the space, under atmospheric or ambient pressure, to form nitrogen oxide (NOx) gas. In other words, air passing through the plasma generation space is ionized when it passes through the concentrated electric field region, and converted into NOx gas consisting of NO₂ gas and NO gas. In this embodiment, microwave energy is used to generate such plasma. The microwave energy is given from the plasma engine 300 to the plasma nozzle 203. The plasma engine 300 to the plasma nozzle 203 will be specifically described later based on FIG. 4 and FIG. 5.

The catalyst section 204 is an catalyst operable to convert NOx gas formed in the plasma nozzle 203, except NO₂ gas, into NO₂ gas. The purification section 205 is operable to purify a sterilizing gas and a reaction product left after the sterilization treatment of the object in each of the first chamber 201 and the second chamber 202. The control section 206 includes a CPU (Central Processing Unit), wherein it is operable, based on a given program, to perform an operational control for the first to third pumps P21 to P23, and an opening/closing control for the first to ninth solenoid valves V21 to V29.

The plasma nozzle 203 and each of the first chamber 201 and the second chamber 202 are communicated with each other via the first to fifth pipes 211 to 215 constituting a circulation path for circulating gas. The plasma nozzle 203 and an inlet side of the first chamber 201 are connected via the first pipe 211 (a part of a first pipe line), and the first solenoid value V21 is installed in the first pipe 211. The second pipe 212 (a part of the first pipe line) is branched from the first pipe 211 at a position upstream of an installation position of the first solenoid valve V21. The second solenoid valve V22 is installed in the second pipe 212. An inlet side of the second chamber 202 is connected to the plasma nozzle 203 via the second pipe 212 and an upstream portion of the first pipe 211.

The first chamber 201 and the second chamber 202 are communicated with each other via the third pipe 213 (second pipe line). The third pipe 213 is used as a supply path for allowing NO₂ gas remaining after performing the sterilization treatment in one of the chambers (residual sterilizing agent) to be introduced into the other chamber. The third solenoid valve V23 is installed in the third pipe 213.

An outlet side of the first chamber 201 and the plasma nozzle 203 are connected via the fourth pipe 214. The fourth solenoid valve V24, the catalyst section 204, and the first pump P21 (a part of the supply device), are installed in the fourth pipe 214 in this order from an upstream side. The fifth pipe 215 has an upstream end provided with the fifth solenoid valve V25 and connected to an outlet side of the second chamber 202, and a downstream end of the fifth pipe 215 is joined with the fourth pipe 214 at a position between the fourth solenoid valve V24 and the catalyst section 204. The first pump P21 is a pump for generating a gas flow directed from the plasma nozzle 203 as an upstream side, toward the first chamber 201 and the second chamber 202 as a downstream side, via the first to fifth pipes 211 to 215.

A charge system and a discharge system are provided to the first chamber 201 and the second chamber 202. The charge system is a system for introducing dry air as a row material gas into each of the first chamber 201 and the second chamber 202, and returning a depressurized inside of each of the first chamber 201 and the second chamber 202 to atmospheric or ambient pressure. The discharge system is a system for depressurizing/drying an inside of each of the first chamber 201 and the second chamber 202, and discharging a sterilizing gas remaining in each of the first chamber 201 and the second chamber 202, while purifying the sterilizing gas.

The charge system includes the sixth pipe 216, the seventh pipe 217 and the second pump P22. The sixth pipe 216 has one end provided with the second pump P22, and the other end connected to the first chamber 201 through the sixth solenoid valve V26. The seventh pipe 217 provided with the seventh solenoid valve V27 is branched from the sixth pipe 216 at a position upstream of the sixth solenoid valve V26, and a downstream end thereof is connected to the second chamber 202. In cases where outside air is introduced via the charge system, it is desirable to provide an air dryer for removing moisture from the air, at a suitable position of the charge system.

The discharge system includes the eighth pipe 218, the ninth pipe 219, the purification section 205 and the third pump P23. The eighth pipe 218 has an upper end connected to the first chamber 201 and a downstream end provided with the third pump P23, and the eighth solenoid valve V28 and the purification section 205 are installed in an intermediate portion thereof. The ninth pipe 219 has an upstream end connected to the second chamber 202, an intermediate portion provided with the ninth solenoid valve V29, and a downstream end joined with the eighth pipe 218 at a position between the eighth solenoid valve V28 and the purification section 205.

The plasma engine 300 and the plasma nozzle 203 will be described below. FIG. 4 is a block diagram schematically showing a structure of the plasma engine 300. The plasma engine 300 is a device designed to generate microwave energy and supplying the microwave energy to the plasma nozzle 203, wherein it includes a microwave generator 301 for generating a microwave, and a waveguide 302 for propagating the microwave. The plasma nozzle 203 is attached to the waveguide 302. Further, an isolator 303, a coupler 304 and a tuner 305 are provided between the microwave generator 301 and the waveguide 302.

The microwave generator 301 includes a microwave generation source, such as a magnetron for generating a microwave having a frequency, for example, of 2.45 GHz, and an amplifier for adjusting a microwave generated by the microwave generation source to have a given output intensity. Preferably, in this embodiment, a continuously-variable microwave generator 301 capable of generating microwave energy, for example, of 1W to 3 kW, is used.

The waveguide 302 is made of a non-magnetic metal, such as aluminum, and formed to have a cross-sectionally-rectangular elongate tubular shape to allow a microwave generated by the microwave generator 301 to be propagated in a longitudinal direction thereof. A sliding short 307 is attached to a distal end of the waveguide 302 through a flange portion 306. The sliding short 307 is a member designed to change a reflection position of a microwave to adjust a standing wave pattern.

The isolator 303 is a device for inhibiting a reflected microwave from entering from the waveguide 302 into the microwave generator 301, wherein it includes a circulator 308 and a dummy load 309. The circulator 308 is operable to direct a microwave generated by the microwave generator 301, toward the waveguide 302, while directing a reflected microwave toward the dummy load 309. The dummy load 309 is operable to absorb the reflected microwave and convert it into heat. The coupler 304 is operable to measure an intensity of microwave energy. The tuner 305 is a device provided with a stab capable of protruding into the waveguide 302 and designed to perform an adjustment for minimizing a reflected microwave, i.e., an adjustment for maximizing consumption of microwave energy by the plasma nozzle 203. The coupler 304 is used in this adjustment.

FIG. 5 is a sectional view showing the plasma nozzle 203 in a state after it is attached to the waveguide 302. The plasma nozzle 203 includes a center conductor 311 (first conductor), an outer conductor 312 (second conductor), a spacer 313 and a protective tube 314.

The center conductor 311 is constructed by a rod-shaped member made of a metal excellent in electrical conductivity, and an upper end 311B thereof protrudes into an inside of the waveguide 302 by a given length. The protruding upper end 311B functions as an antenna portion for receiving a microwave propagated through the waveguide 302.

The outer conductor 312 is a tubular body made of a metal excellent in electrical conductivity and formed to have a columnar space 312H (plasma generation space) for receiving therein the center conductor 311. The center conductor 311 is disposed on a central axis of the columnar space 312H. The outer conductor 312 is fixed to the waveguide 302 in such a manner that it is fitted into a tubular-shaped metal flange plate 321 integrally attached to a bottom plate of the waveguide 302, and fastened by a screw 322. The waveguide 302 is set to a ground potential, and thereby the outer conductor 213 is also set to the ground potential.

The outer conductor 312 also has a gas supply hole 312N penetrating to the columnar space 312H through the outer peripheral wall thereof. A downstream side of the fourth pipe 214 is connected to the gas supply hole 312N. Further, an upstream end of the first pipe 211 is connected to a lower end of the columnar space 312H. This allows air introduced into each of the first chamber 201 and the second chamber 202 to be routed through the columnar space 312H.

The spacer 313 holds the center conductor 311 and seals between an internal space of the waveguide 302 and the columnar space 312H. For example, a thermally-resistant resin material, such as polytetrafluoroethylene, or an insulating member made of a ceramics, may be used for the spacer 313. A stepped portion is provided in an upper end region of the columnar space 312H of the outer conductor 312, and the spacer 313 is supported by the stepped portion. The center conductor 311 held by the spacer 313 is insulated from the outer conductor 312. The protective tube 314 is constructed by a silica glass pipe having a given length, and fitted into a lower end region of the columnar space 312H to prevent an abnormal discharge (arcing) in a lower edge 321T of the outer conductor 312.

In the plasma nozzle 203 constructed as above, when the center conductor 311 receives a microwave propagated through the waveguide 302, a potential difference occurs between the center conductor 311, and the outer conductor 312 having the ground potential. Particularly, a concentrated electric field region is formed in a vicinity of a lower end 311T of the center conductor 311 and the lower edge 312T of the outer conductor 312. In this state, when a gas (air) containing oxygen molecules and nitrogen molecules is supplied from the gas supply hole 312N into the columnar space 312H, the gas is excited, so that plasma (ionized gas) is generated in the vicinity of the lower end 311T of the center conductor 311. The plasma includes NOx and free radicals. Further, this plasma is reactive plasma having a gas temperature close to outside temperature although an electron temperature thereof is several tens of thousands of degrees (the reactive plasma has an extremely high electron temperature indicated by electrons therein, as compared with a gas temperature indicated by neutrons therein), and plasma to be generated under normal pressure.

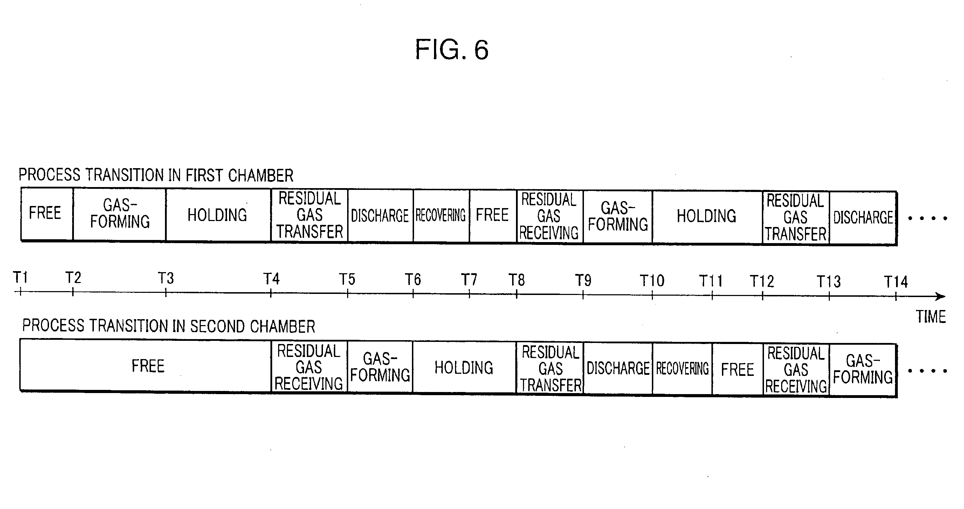

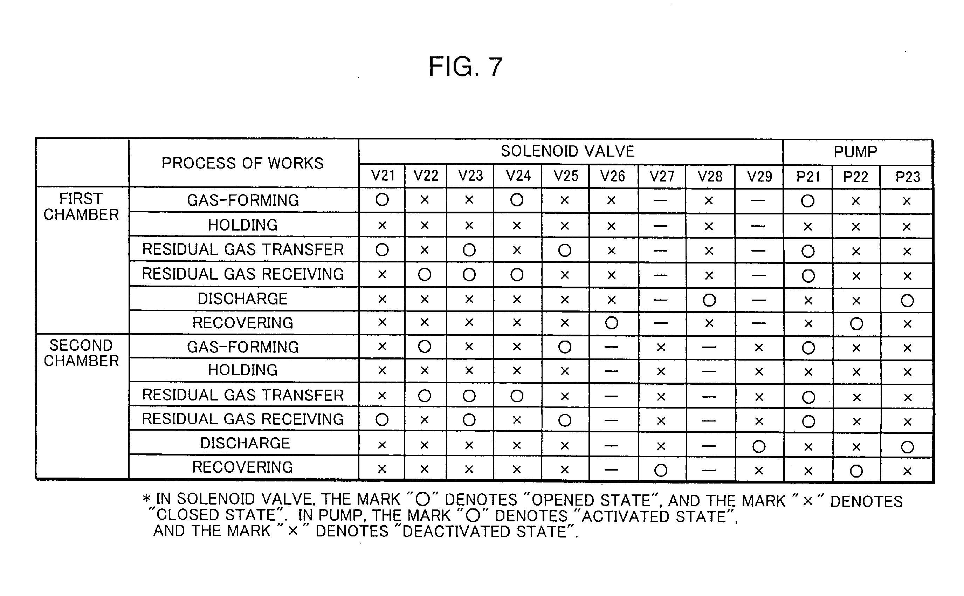

An operation of the sterilizer 200 according to the second embodiment constructed as above will be described. FIG. 6 is a time chart showing the operation of the sterilizer 200. FIG. 7 is a tabular diagram showing a control state of the solenoid valves V21 to V29 and the pump P21 to P23. An operation mode (step) of the sterilizer 200 roughly comprises the following five types.
(1) Gas-forming step of circulating air or a mixed gas of air and a residual sterilizing gas, introduced into each of the first chamber 201 and the second chamber 202, through the plasma nozzle 203, to form NO₂ gas (sterilizing gas) having a given concentration.
(2) Holding step of hermetically closing the first chamber 201 or the second chamber 202 filled with the NO₂ gas having the given concentration, and holding this state only for a given time period required for sterilization to allow the NO₂ gas to act on an object so as to perform a sterilization treatment.
(3) Residual-sterilizing-gas transfer or receiving step of circulating gas while communicating between the first chamber 201 and the second chamber 202, to introduce NO₂ gas remaining after performing the sterilization treatment in one of the chambers, into the other chamber.
(4) Discharge step of discharging gas remaining in the chamber from which a residual sterilizing gas has been transferred, while purifying the gas. In this step, the chamber is placed in a vacuum state.
(5) Recovering step of introducing air into the chamber placed in the vacuum state in the discharge step, to provide atmospheric or ambient pressure therein. The air introduced in this step serves as a raw material gas in a next gas-forming step.

The step names in each of the chambers described in the time chart of FIG. 6 are in corresponding relation to the respective step names described in FIG. 7. In FIG. 7, which of the solenoid valves V21 to V29 is opened or closed in each of the steps, and which of the pumps P21 to P23 is activated or deactivated in each of the steps, are expressed by the mark "O" and the mark "x". Thus, in the following description, explanation in writing about the opened/closed state of the solenoid valve and the operational state of the pump will be omitted.

In FIG. 6, between a time T1 and a time T2, the first chamber 201 is in a free period where a door (not shown) thereof can be opened and closed. In this free period, a user sets an un-sterilized object in the chamber, or takes out a sterilized object from the chamber.

At the time T2, an inside of the first chamber 201 has atmospheric or ambient pressure, and air exists therein. At the time T2, in order to allow the sterilizer 200 to perform the gas-forming step, the control section 206 controls the solenoid valves V21 to V29 and the pumps P21 to P23 in a manner as shown in FIG. 7, and operates to activate the plasma engine. Thus, a loop-like closed space comprising the plasma nozzle 203 (columnar space 312H), the first pipe 211, the first chamber 201 and the fourth pipe 214, is formed, and air is circulated through the closed space.

The air repeatedly passes through the columnar space 312H of the plasma nozzle 203 which is in an activated state according to microwave energy given thereto, so that the air is plasmatized (ionized) and converted into NOx gas. This NOx gas is converted into NO₂ gas through the catalyst section 204. Along with the continuous circulation of the air, a concentration of NO₂ in the closed space will be gradually increased.

When it is detected at a time T3 that NO₂ gas having a given concentration required for sterilization resides in the first chamber 201, the control section 206 operates to perform the holding step. Through this operation, the NO₂ gas and the object contact each other in the hermetically-closed first chamber 201. The holding step will be continued until a time T4, with a time interval required for sufficient sterilization of the object. The second chamber 202 is in a free period until the time 4. In this free period, the user sets an un-sterilized object in the second chamber 202.

At the time T4, the control section 206 operates to perform the residual-sterilizing-gas transfer or receiving step of transferring a residual sterilizing gas from the first chamber 201 to the second chamber 202. In FIG. 7, the "residual gas transfer (receiving)" in the first chamber 201, and the "residual gas transfer (receiving)" in the second chamber 202, are the same operation mode. Through this operation, a loop-like closed space comprising the first pipe 211, the first chamber 201, the third pipe 213, the second chamber 202, the fifth pipe 215, the fourth pipe 214 and the plasma nozzle 203 is formed, and NO₂ gas existing in the first chamber 201 is circulated through the closed space. After this circulation is continued to some extent, respective NO₂ concentrations in the first chamber 201 and the second chamber 202 become equal to each other, and consequently a part of air in the second chamber 202 is substituted with NO₂ gas (time T5).

At the time T5, the control section 206 operates to perform the discharge step for the first chamber 201, and concurrently perform the gas-forming step for the second chamber 202. Thus, gas remaining in the first chamber 201 is discharged via the eighth pipe 218. Concurrently, a loop-like closed space comprising the plasma nozzle 203, the first pipe 211, the second pipe 212, the second chamber 202, the fifth pipe 215 and the fourth pipe 214, is formed, and a mixed gas of air and NO₂ gas is circulated through the closed space.

In this circulation state, when the plasma nozzle 203 is activated, an NO₂ concentration in the closed space will be gradually increased. Then, at a time T6, NO₂ gas having a given concentration required for sterilization resides in the second chamber 202. In this process, a part of air in the second chamber 202 has already been substituted with NO₂ gas, and thereby a time period between the time T5 and the time T6 can be fairly shortened as compared with a time period between the time T2 and the time T3 for the initial gas-forming step in the first chamber 201. In practice, a time period (between the time T4 and the time T5) of the residual-sterilizing-gas transfer/receiving can be actually set to a short time period. Thus, a time period required for forming a sterilizing gas in the second chamber 202 can be significantly shortened.

At the time T6, the control section 206 operates to perform the recovery step for the first chamber 201, and concurrently perform the holding step for the second chamber 202. Thus, air is introduced from the sixth pipe 216 into the first chamber 201, and the first chamber 201 recovers to atmospheric or ambient pressure in a purified state at a time T7. After that, the first chamber 201 enters in a free period. In this free period, the user can take out the sterilized object from the first chamber 201 and set a new object therein. On the other hand, in the second chamber 202, the sterilization treatment of the object will be continued until a time T8.

At the time T8, the control section 206 operates to perform the gas transfer/receiving step of transferring a residual NO₂ gas from the second chamber 202 to the first chamber 201. Thus, a loop-like closed space comprising the first pipe 211, the second pipe 212, the second chamber 202, the third pipe 213, the first chamber 201, the fourth pipe 214 and the plasma nozzle 203 is formed, and NO₂ gas existing in the second chamber 202 is circulated through the closed space. After this circulation is continued to some extent, respective NO₂ concentrations in the first chamber 201 and the second chamber 202 become equal to each other, and consequently a part of air in the first chamber 201 is substituted with NO₂ gas (time T9).

At the time T9, the control section 206 operates to perform the discharge step for the second chamber 202, and concurrently perform the gas-forming step for the first chamber 201. Thus, gas remaining in the second chamber 202 is discharged via the ninth pipe 219. Concurrently, an NO₂ gas concentration in the first chamber 201 will be gradually increased in the same manner as that in the period between the time T2 and the time T3. As with the aforementioned operation, a part of air in the first chamber 201 has already been substituted with NO₂ gas by the last gas transfer, and thereby a time period required for forming a sterilizing gas in the first chamber 201 can be significantly shortened.

At a time T10 when the NO₂ gas concentration in the first chamber 201 reaches a given value, the control section 206 operates to perform the holding step for the first chamber 201, and concurrently perform the recovering step for the second chamber 202. Thus, air is introduced into the second chamber 202 via the sixth pipe 216 and the seventh pipe 217, and the second chamber 202 recovers to atmospheric or ambient pressure in a purified state at a time T11. After that, the second chamber 202 enters in a free period. In this free period, the user can take out the sterilized object from the second chamber 202 and set a new object therein. On the other hand, in the first chamber 201, the sterilization treatment of the second object will be continued until a time T12. After the time T12, the same process as that after the time T4 will be repeated.

In the sterilizer 200 according to the second embodiment as described above, an NO₂ gas after the sterilizing treatment is exchanged between the first chamber 201 and the second chamber 202, so that a time period of the NO₂ gas-forming step can be shortened to improve operating efficiency of the sterilization treatment.

### [THIRD EMBODIMENT]

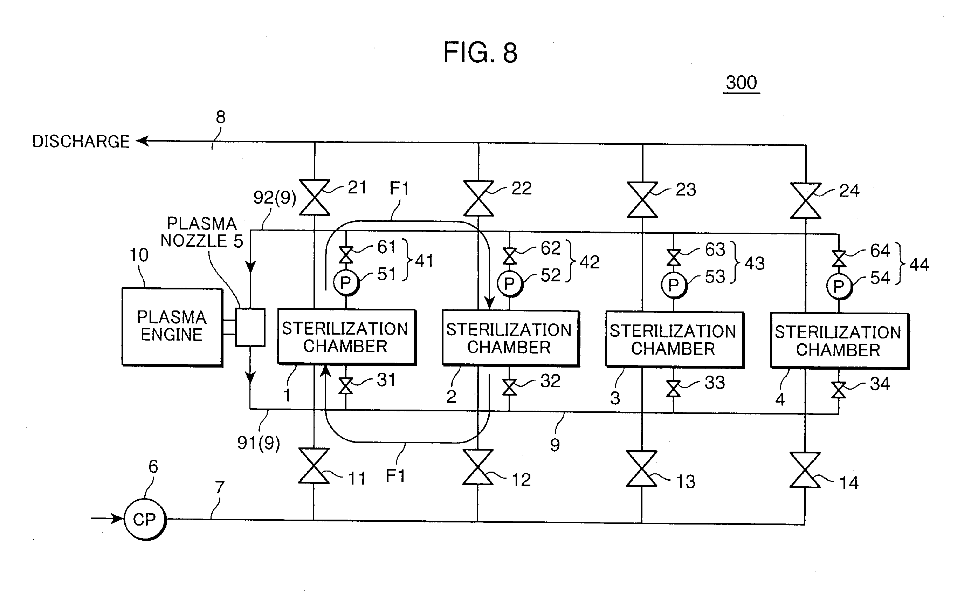

FIG. 8 is a block diagram showing a sterilizer 300 according to a third embodiment of the present invention. This sterilizer 300 includes first to fourth sterilization chambers 1 to 4, a plasma nozzle 5 (supply source for a sterilizing agent), a compressor 6, a charge pipe line 7, a discharge pipe line 8, a circulation pipe line 9 (serving as both a first pipe line and a second pipe line), and a plasma engine 10.

The sterilizer 300 is an apparatus designed to allow each of the first to fourth sterilization chambers 1 to 4 to be filled with a sterilizing gas (gas, as a product by a plasma reaction, containing oxygen radicals, ozone, NOx, etc.) formed by the plasma nozzle 5, in a state after an object is placed in each of the sterilization chambers 1 to 4, so as to perform a sterilization treatment of the objects in the sterilization chambers 1 to 4 in a parallel (concurrent) or successive manner.

Each of the first to fourth sterilization chambers 1 to 4 is a chamber capable of receiving therein an object, as with the chambers 101, 102 illustrated in the first and second embodiments. Although the third embodiment shows four sterilization chambers as an example, the sterilizer may be formed in a structure having a larger number of sterilization chambers.

The plasma nozzle 5 is operable to plasmatize (ionize) a raw material gas under atmospheric or ambient pressure to form a sterilizing gas. This plasma nozzle 5 has the same structure as that of the plasma nozzle 203 illustrated in the second embodiment, i.e., a structure adapted to generate plasma according to energy given from the plasma engine 10.

The compressor 6 is provided to send air to each of the sterilization chambers 1 to 4 to dry and discharge gas remaining in the chamber.

The charge pipe line 7 and the discharge pipe line 8 are laid for drying and degassing of the first to fourth sterilization chambers 1 to 4. The charge pipe line 7 is branched in parallel with respect to respective ones of the sterilization chambers 1 to 4 to introduce outside air into each of the sterilization chambers 1 to 4. The compressor 6 is installed in a starting end of the charge pipe line 7, and openable/closable valves 11 to 14 each constructed of a solenoid value or the like is provided in respective ones of the parallel pipes. Similarly, the discharge pipe line 8 is branched in parallel with respect to respective ones of the first to fourth sterilization chambers 1 to 4 to discharge gas remaining in each of the sterilization chambers 1 to 4. The discharge pipe line 8 has openable/closable valves 21 to 24 provided in respective ones of the parallel pipes thereof.

The circulation pipe line 9 is a pipe line laid to extend from the plasma nozzle 5 and in parallel with respect to respective ones of the first to fourth sterilization chambers 1 to 4, to circulate a sterilizing gas formed in the plasma nozzle 5. The circulation pipe line 9 includes a first circulation pipe line 91 arranged on a side for introducing a sterilizing gas into each of the sterilization chambers 1 to 4, and formed in a manifold structure, and a second circulation pipe line 92 arranged on a side for leading out a sterilizing gas from each of the sterilization chambers 1 to 4, and formed in a manifold structure in the same manner. The circulation pipe line 9 is a pipe line which is also used when a residual sterilizing gas is exchanged between the first to fourth sterilization chambers 1 to 4.

The first circulation pipe line 91 has openable/closable valves 31 to 34 provided in branched portions thereof, respectively. The second circulation pipe line 92 has a supply mechanism 41 to 44 provided in branched portions thereof, respectively. Each of the supply mechanism 41 to 44 includes a pump (51 to 54) for generating a gas flow in the circulation pipe line 9, and an openable/closable valve (61 to 64) comprised of a solenoid valve or the like.

The sterilizer 300 includes a control unit (not shown). The control unit is operable to drive-control the plasma engine 10, the compressor 6, the pumps 51 to 54, and the openable/closable valves 11 to 14, 21 to 24, 31 to 34, 61 to 64.

The above third embodiment is one example where the drying and discharge of gas remaining in each of the sterilization chambers 1 to 4 is performed using fresh outside air. For example, in cases where a high-pressure gas source is connected to the charge pipe line 7 in an embodiment using an inert gas in the drying and discharge, the compressor 6 may not be particularly provided. Further, for example, high-pressure steam, EOG (Ethylene Oxide Gas), formalin or hydrogen peroxide may be used in place of the sterilizing gas formed by a plasma reaction. In cases where a harmful gas, such as ozone, NOx, EOG or formalin, is used as a sterilizing agent, a collection device for such a gas is provided in the discharge pipe line 8.

An operation of the sterilizer 300 constructed as above will be described. Before starting a sterilization treatment, the openable/closable valves 11 to 14, 21 to 24 of the charge pipe line 7 and the discharge pipe line 8 are opened, and the openable/closable valves 31 to 34, 61 to 64 installed in the circulation pipe line 9 are closed. In this state, the compressor 6 is driven to perform the drying and discharge of gas remaining in the sterilization chambers 1 to 4 using fresh outside air. Then, an object is placed in each of the sterilization chambers 1 to 4.

After placing the object, the openable/closable valves 11 to 14, 21 to 24 are closed, and the openable/closable valves 31, 61 (a first shutoff device) of the circulation pipe line 9 are firstly opened. Thus, the first sterilization chamber 1 and the plasma nozzle 5 (plasma generation space) are set to a communicated state. In this state, when the plasma engine 10 is operated, and the pump 51 is activated, a sterilizing gas is formed by a plasma reaction occurring in the plasma nozzle 5, and a sterilizing gas concentration in the first sterilization chamber 1 will be gradually increased. In this manner, the object in the first sterilization chamber 1 is subjected to a sterilization treatment.

When the sterilization in the first sterilization chamber 1 is completed, a sterilizing gas remaining in the first sterilization chamber 1 is introduced into each of the remaining sterilization chambers 2 to 4 without immediately performing the discharge of gas remaining in the first sterilization chamber 1. For example, in cases where the residual sterilizing gas is introduced into the second sterilization chamber 2, the control unit operates to open the openable/closable valves 31, 32, 61, 62, and then drive the pumps 51, 52.

Thus, as indicated by the arrowed line F1 in FIG. 8, the first sterilization chamber 1 where the sterilization treatment has been completed, and the second sterilization chamber 2 where a sterilization treatment will be performed from now, are set to a communicated state to allow gas exchange to be performed between the two sterilization chambers 1, 2. Specifically, a used sterilizing gas in the first sterilization chamber 1 next to the second sterilization chamber 2 is preliminarily mixed with fresh outside air initially filled in the second sterilization chamber 2 where a sterilization treatment will be performed next. On an assumption that each volumes of the sterilization chambers 1, 2 are equal to each other, a sterilizing gas having a concentration of about 50% will reside in the second sterilization chamber 2.

At a timing when respective sterilizing gas concentrations in the first and second sterilization chambers 1, 2 are counterbalanced, the control unit operates to close the openable/closable valve 31, and open the openable/closable valves 11, 21, and then operates to drive the compressor 6 to discharge gas remaining in the first sterilization chamber 1. Concurrently, the control unit operates to activate the pump 52 and the plasma engine 10 to increase a sterilizing gas concentration in the second sterilization chamber 2, while keeping each of the openable/closable valves 32, 62 (a second shutoff device) in an opened state. In this process, a time period to increase the concentration to a required value for the sterilization treatment can be shortened, because a part of air in the second sterilization chamber 2 has already been substituted with the sterilizing gas. This is particularly effective because, when a plasma reaction is used for forming a sterilizing gas, the sterilizing-gas formation is liable to require a certain amount of time. In addition, an amount of the sterilizing gas to be consumed can be reduced by reusing a used sterilizing gas. Further, in cased where the sterilizing agent is ozone or the like, harmful gas to be discharged can be reduced.

After completing the sterilization treatment in the second sterilization chamber 2, gas is exchanged between the second sterilization chamber 2 and the third sterilization chamber 3, in the same way. For this exchange, the control unit operates to open the openable/closable valves 32, 33, 62, 63, and then drive the pumps 52, 53. Thus, a sterilizing gas used in the second sterilization chamber 2 next to the third sterilization chamber 3 is preliminarily mixed with air filled in the third sterilization chamber 3 where a sterilization treatment will be performed next. Then, the step of increasing a sterilizing agent concentration in the third sterilization chamber 3 is performed in the same manner as described above. In this step, a time period required for increasing the concentration up to a required value for the sterilization treatment can be shortened because a part of air in the third sterilization chamber 3 has already been substituted with a sterilizing gas. After that, gas in the second sterilization chamber 2 is discharged, and concurrently the sterilization treatment is performed in the third sterilization chamber 3.

After completing the sterilization treatment in the third sterilization chamber 3, gas exchange between the third sterilization chamber 3 and the fourth sterilization chamber 4 will be performed in the same way. Subsequently, gas exchange between the fourth sterilization chamber 4 and the first sterilization chamber 1 will be performed in the same way. In this manner, the sterilization treatments are sequentially performed.

The transfer mode is not limited to the above manner where a residual sterilizing gas is transferred from one of a plurality of sterilization chambers to one of the remaining sterilization chambers, but may be configured such that a residual sterilizing gas is transferred from one or more of a plurality of sterilization chambers to two or more of the remaining sterilization chambers. For example, after completing a sterilization treatment in the first sterilization chamber 1, a residual sterilizing gas in the first sterilization chamber 1 may be introduced into the second sterilization chamber 2 and the third sterilization chamber 3. Alternatively, it may be configured such that a sterilization treatment is performed in the first and second sterilization chambers 1, 2 in a parallel manner, and, after completing the sterilization treatment, a residual sterilizing gas in the first and second sterilization chambers 1, 2 is introduced into the third and fourth sterilization chambers 3, 4.

Each of the pumps 51 to 54 is a pump to be used for gas circulation between the sterilization chambers. In cases where there is a certain level of pressure difference between a sterilization chamber in a post-treatment state and a sterilization chamber in a pre-treatment state, gas exchange between the sterilization chambers can be achieved only by appropriately opening the openable/closable valves 31 to 34, 61 to 64. In such cases, the pumps 51 to 54 may not be particularly provided. Further, when gas exchange is performed between the first and second sterilization chambers 1, 2, only one 51 of the pumps may be driven without driving the other pump 52. Alternatively, the pumps 51, 52 may be activated to forcedly introduce gas of the first sterilization chamber 1 into the second sterilization chamber 2 so as to increase a sterilizing gas concentration in the second sterilization chamber 2.

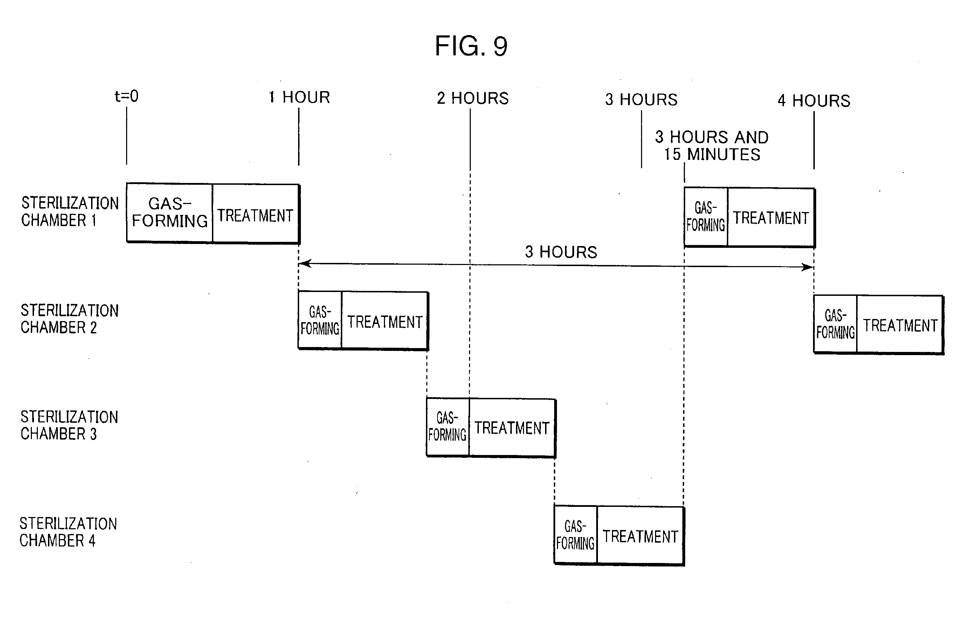

FIG. 9 is a diagram for explaining an example of an actual usage of the sterilizer 300 according to the third embodiment. FIG. 9 shows an example where the same sterilization treatment is sequentially performed in the sterilization chambers 1 to 4 of the sterilizer 300 in a successive and repetitive manner. An object in this example is a medical product, a sanitary product or the like which has a need for successively subjecting a large number of products to a sterilization treatment.

In the example of FIG. 9, a volume of each of the sterilization chambers 1 to 4 was set to 100 liters, wherein a time period required for forming a sterilizing gas having a concentration required for a sterilization treatment, by the plasma nozzle 5, was set to 30 minutes, and a time period required for an actual sterilization treatment after each of the sterilization chambers 1 to 4 is filled with the sterilizing gas was set to 30 minutes.

In the first sterilization chamber 1 where a first sterilization treatment will be performed, it needs to take one hour for a total process for sterilizing gas formation and sterilization treatment. However, in the remaining sterilization chambers, a used sterilizing gas in the sterilization chamber of a preceding stage is introduced into the sterilization chamber of subsequent stage, so that a time period for the sterilizing gas formation becomes about one-half. Thus, a time period required for a second or subsequent sterilization treatment in each of the sterilization chambers 2, 3, 4, 5 becomes about 45 minutes, and one process cycle for the first to fourth sterilization chambers 1 to 4 can be completed within 3 hours and 15 minutes in total. If the process is continued subsequently, a process time in the first sterilization chamber 1 also becomes 45 minutes, and a cycle time of the process can be reduced to 3 hours.

In the sterilizer 300 according to the third embodiment, after completing the sterilization treatment in the first sterilization chamber 1, a sterilizing gas is charged in the second sterilization chamber 2 in which a sterilization treatment will be performed next, so that a counterpart for utilizing a used sterilizing gas can be continuously ensured, and a time period required for the sterilizing gas formation, can be shortened in all of the first to fourth sterilization chambers 1 to 4, while facilitating an reduction in amount of the sterilizing gas to be consumed. Further, in practice, it is also necessary to ensure a time period for carrying an object in/out of a sterilization chamber, although it is not taken into account in the above description. In cases where a sterilization treatment is successively performed as shown in FIG. 9, a plurality of small-size sterilization chambers 1 to 4 may be used. In this case, the carry-in/out time period can be deconcentrated to prevent prolonged interruption in the treatment which would otherwise occur in use of a large-size sterilization chamber.

The aforementioned specific embodiments primarily include an invention having the following constructions.

A sterilizer according one aspect of the present invention includes: a supply source for a sterilizing agent; a first sterilization chamber and a second sterilization chamber each adapted to be filled with the sterilizing agent while placing an object therein so as to subject the object to a sterilization treatment; a first pipe line connecting the supply source and each of the first sterilization chamber and the second sterilization chamber; a second pipe line connecting the first sterilization chamber and the second sterilization chamber; and a supply mechanism adapted to allow a residual part of the sterilizing agent used for the sterilization treatment in the first sterilization chamber to be introduced into the second sterilization chamber via the second pipe line.

In the above sterilizer, after the sterilization treatment is performed in the first sterilization chamber, a residual sterilizing agent in the first sterilization chamber is introduced into the second sterilization chamber by the supply mechanism. Thus, a used sterilizing gas in the first sterilization chamber is preliminarily mixed with gas in the second sterilization chamber. This makes it possible to shorten a time period required for filling the second sterilization chamber with the sterilizing agent, and reduce an amount of the sterilizing agent to be consumed.

Preferably, the above sterilizer further includes a introduction mechanism adapted, after the residual sterilizing agent is introduced into the second sterilization chamber, to allow the sterilizing agent to be further introduced from the supply source into the second sterilization chamber via the first pipe line. In this sterilizer, if a sufficient sterilizing agent cannot be obtained only by the residual sterilizing agent introduced into the second sterilization chamber, a required amount of the sterilizing agent can be supplied to the second sterilization chamber by the introduction mechanism.

The above supply source may include a sterilizing gas-forming section adapted to form a sterilizing gas as the sterilizing agent, based on a plasma reaction. The sterilizing-gas formation based on a plasma reaction is liable to require a certain amount of time. For this reason, a used sterilizing gas in the first sterilization chamber is diverted to a sterilizing gas for the second sterilization chamber, which is effective because a time period required for charging a sterilizing gas in the second sterilization chamber in a required amount for the sterilization treatment can be significantly shortened.

Preferably, in the above sterilizer, the sterilizing gas-forming section has a first electrode, a second electrode, a plasma generation space defined between the first and second electrodes, and a plasma engine operable to give energy between the first and second electrodes to generate an electric field, and the first pipe line includes a circulation path which enters from the plasma generation space into each of the first sterilization chamber and the second sterilization chamber, and then returns from each of the first sterilization chamber and the second sterilization chamber to the plasma generation space.

In this sterilizer, a raw material gas is plasmatized (ionized) in the plasma generation space, while circulating the raw material gas in the circulation path, so that a sterilizing gas concentration can be gradually increased.

Preferably, the above sterilizer further includes: a first shutoff device adapted to shut off the first sterilization chamber from the circulation path; a second shutoff device adapted to shut off the second sterilization chamber from the circulation path; a circulation mechanism adapted to generate a gas flow in the circulation path; and a control device adapted to control the first and second shutoff device and the circulation mechanism, wherein the control device is operable to perform: a first control of, under a condition that the second sterilization chamber is shut off from the circulation path by the second shutoff device, activating the circulation mechanism to circulate a raw material gas between the plasma generation space and the first sterilization chamber so as to fill the first sterilization chamber with a sterilizing gas having a given concentration; and a second control of, under a condition that the first sterilization chamber is shut off from the circulation path by the first shutoff device, after a residual sterilizing gas is introduced from first sterilization chamber into the second sterilization chamber by the supply mechanism, activating the circulation mechanism to circulate a mixed gas of a raw material gas and the residual sterilizing gas between the plasma generation space and the second sterilization chamber so as to fill the second sterilizing chamber with a sterilizing gas having a given concentration.

This sterilizer can perform a process of firstly forming a sterilizing gas in a circulation space routed through the first sterilization chamber to perform the sterilization treatment in the first sterilization chamber, then transferring a residual sterilizing gas in the first sterilization chamber to the second sterilization chamber, and subsequently forming a sterilizing gas in a circulation space routed through the second sterilization chamber to perform the sterilization treatment in the second sterilization chamber. Thus, the sterilization treatment of the objects can be successively performed while offsetting respective sterilization treatment timings in the first sterilization chamber and the second sterilization chamber. This makes it possible to continuously ensure a counterpart for utilizing a used sterilizing gas, and facilitate shortening the time period required for the sterilization treatment and reducing an amount of the sterilizing agent, in all of the sterilization chambers.

Preferably, in the above sterilizer, the supply mechanism is a third shutoff device installed in the second pipe line and adapted to shut off the second pipe line; and the control device is adapted to control an operation of the third shutoff device, wherein the control device is operable, between the first control and the second control, to perform a third control of releasing the respective shutoff states by the first to third shutoff device, and activating the circulation mechanism to circulate the residual sterilizing gas between the plasma generation space and each of the first sterilization chamber and the second sterilization chamber. In this sterilizer, the transfer of the residual sterilizing gas from the first sterilization chamber to the second sterilization chamber can be achieved using the circulation path:

Preferably, in the above sterilizer, the second pipe line forms a part of the circulation path, and the control device is operable, between the first control and the second control, to perform a fourth control of releasing the respective shutoff states by the first and second shutoff device, and activating the circulation mechanism to circulate the residual sterilizing gas between the first sterilization chamber and the second sterilization chamber. In this sterilizer, in addition to using the circulation path to achieve the transfer of the residual sterilizing gas from the first sterilization chamber to the second sterilization chamber, a part of the circulation path can be used as the second pipe line, which makes it possible to simplify a pipe line configuration.

In the above sterilizer, the object may be a medical product or a sanitary product. These articles have a need for successively subjecting a large number of the articles to a sterilization treatment. Thus, the above shortening of the time period is particularly effective.

A sterilization treatment method according to another aspect of the present invention is a method for a sterilization treatment of an object, using a supply source for a sterilizing agent, and a first sterilization chamber and a second sterilization chamber each adapted to be filled with the sterilizing agent. The method includes the steps of: supplying the sterilizing agent from the supply source into the first sterilization chamber; performing the sterilization treatment of the object in the first sterilization chamber; introducing a residual part of the sterilizing agent used for the sterilization treatment in the first sterilization chamber; supplying the sterilizing agent from the supply source into the second sterilization chamber; and performing the sterilization treatment of the object in the second sterilization chamber.

In this method, after the sterilization treatment is performed in the first sterilization chamber, a residual sterilizing agent in the first sterilization chamber is introduced into the second sterilization chamber. This makes it possible to shorten a time period required for filling the second sterilization chamber with the sterilizing agent, and reduce an amount of the sterilizing agent to be consumed. In addition, the sterilization treatment of the objects can be successively performed while offsetting respective sterilization treatment timings in the first sterilization chamber and the second sterilization chamber.

## Claims

1. A sterilizer comprising:
a supply source for a sterilizing agent;
a first sterilization chamber and a second sterilization chamber each adapted to be filled with the sterilizing agent while placing an object therein so as to subject the object to a sterilization treatment;
a first pipe line connecting the supply source and each of the first sterilization chamber and the second sterilization chamber;
a second pipe line connecting the first sterilization chamber and the second sterilization chamber; and
a supply mechanism adapted to allow a residual part of the sterilizing agent used for the sterilization treatment in the first sterilization chamber to be introduced into the second sterilization chamber via the second pipe line.

2. The sterilizer as defined in claim 1, which further comprises a introduction mechanism adapted, after the residual sterilizing agent is introduced into the second sterilization chamber, to allow the sterilizing agent to be further introduced from the supply source into the second sterilization chamber via the first pipe line.

3. The sterilizer as defined in claim 1 or 2, wherein the supply source includes a sterilizing gas-forming section adapted to form a sterilizing gas as the sterilizing agent, based on a plasma reaction.

4. The sterilizer as defined in claim 3, wherein:
the sterilizing gas-forming section has a first electrode, a second electrode, a plasma generation space defined between the first and second electrodes, and a plasma engine operable to give energy between the first and second electrodes to generate an electric field; and
the first pipe line includes a circulation path which enters from the plasma generation space into each of the first sterilization chamber and the second sterilization chamber, and then returns from each of the first sterilization chamber and the second sterilization chamber to the plasma generation space.

5. The sterilizer as defined in claim 4, which further comprises:
a first shutoff device adapted to shut off the first sterilization chamber from the circulation path;
a second shutoff device adapted to shut off the second sterilization chamber from the circulation path;
a circulation mechanism adapted to generate a gas flow in the circulation path; and
a control device adapted to control the first and second shutoff device and the circulation mechanism,
wherein the control device is operable to perform:
a first control of, under a condition that the second sterilization chamber is shut off from the circulation path by the second shutoff device, activating the circulation mechanism to circulate a raw material gas between the plasma generation space and the first sterilization chamber so as to fill the first sterilization chamber with a sterilizing gas having a given concentration; and
a second control of, under a condition that the first sterilization chamber is shut off from the circulation path by the first shutoff device, after a residual sterilizing gas is introduced from first sterilization chamber into the second sterilization chamber by the supply mechanism, activating the circulation mechanism to circulate a mixed gas of a raw material gas and the residual sterilizing gas between the plasma generation space and the second sterilization chamber so as to fill the second sterilizing chamber with a sterilizing gas having a given concentration.

6. The sterilizer as defined in claim 4, wherein:
the supply mechanism is a third shutoff device installed in the second pipe line and adapted to shut off the second pipe line; and
the control device is adapted to control an operation of the third shutoff device, the control device being operable, between the first control and the second control, to perform a third control of releasing the respective shutoff states by the first to third shutoff device, and activating the circulation mechanism to circulate the residual sterilizing gas between the plasma generation space and each of the first sterilization chamber and the second sterilization chamber.

7. The sterilizer as defined in claim 4, wherein:
the second pipe line forms a part of the circulation path; and
the control device is operable, between the first control and the second control, to perform a fourth control of releasing the respective shutoff states by the first and second shutoff device, and activating the circulation mechanism to circulate the residual sterilizing gas between the first sterilization chamber and the second sterilization chamber.

8. The sterilizer as defined in any one of claims 1 to 7, wherein the object is a medical product or a sanitary product.

9. A method for a sterilization treatment of an object, using a supply source for a sterilizing agent, and a first sterilization chamber and a second sterilization chamber each adapted to be filled with the sterilizing agent, comprising the steps of:
supplying the sterilizing agent from the supply source into the first sterilization chamber;
performing the sterilization treatment of the object in the first sterilization chamber;
introducing a residual part of the sterilizing agent used for the sterilization treatment in the first sterilization chamber;
supplying the sterilizing agent from the supply source into the second sterilization chamber;
and
performing the sterilization treatment of the object in the second sterilization chamber.
